# EUROPEAN PATENT SPECIFICATION

(11) **EP 0 521 733 B1**
(45) Date of publication and mention of the grant of the patent: **18.09.1996**
(21) Application number: 92306176.6
(22) Date of filing: 03.07.1992
(51) Int. Cl.: G01N 33/49, B01D 21/26

(54) **Serum:plasma separator and tube containing the same**
Serum/Plasma-Separator und Rohr diesen enthaltend
Séparateur de sérum/plasma et tube le contenant

(30) Priority: 05.07.1991 JP 191002/91; 20.09.1991 JP 268746/91
(43) Date of publication of application: 07.01.1993
(73) Proprietor: TOYO INK MANUFACTURING CO., LTD., Tokyo (JP)
(72) Inventor: Satake, Sunao, c/o Toyo Ink Manuf. Co., Ltd., Chuo-ku, Tokyo (JP)
(74) Representative: Woods, Geoffrey Corlett

(56) References cited:
- EP-A- 0 384 331
- US-A- 3 920 557
- US-A- 4 083 784
- US-A- 4 457 782
- US-A- 4 534 798
- US-A- 4 751 001

## Description

The present invention relates to a serum:plasma separator and a tube for the separation of serum:plasma from clot:hemocytes (to be referred to as "blood separation tube" hereinafter). More specifically, it relates to a serum:plasma separator which forms a partition having a medium specific gravity between the serum and clot or between the plasma and hemocytes when the blood is separated into these two components on the basis of a specific gravity difference thereby to facilitate the blood separation procedures. It also relates to a blood separation tube formed by placing the serum:plasma separator in a one end-closed tube and closing the other end of the tube.

A serum:plasma separator used for conventional blood separation procedures is obtained by incorporating a specific gravity or viscosity adjusting aid such as inorganic fine particles of silica or clay, or an organic gelling agent into a resin selected from a liquid silicone resin, chlorinated polybutene, polyisobutene, an acryl polymer and α-olefin/maleic acid diester polymer as main components. The resin used as a main component basically has a hydrophobic nature. The inorganic fine particles increase the viscosity of the serum:plasma separator and impart the serum;plasma separator with a thixotropic nature. The organic gelling agent increases the viscosity of the serum:plasma separator without imparting it with a thixotropic nature.

However, when the above conventional serum:plasma separator is used for centrifugal separation, hemocytes are liable to remain above the serum:plasma separator, i.e., in serum. That is, the separation function of the above conventional serum:plasma separator is insufficient. Further, while centrifugally separated components are stored in a separation tube, migration of components having a concentration gradient through the serum:plasma separator takes place between clot and serum or between hemocytes and plasma. Therefore, values obtained by measuring the centrifugally separated and stored blood components, such as potassium ion value, are unreliable.

Meanwhile, the resin used as a main component is liquid-like. When a both end-closed tube containing the serum;plasma separator is stored by placing it horizontally, unpractically, the serum:plasma separator flows. In order to overcome this effect, a thixotropic aid such as finely milled mica or colloidal silica or an organic gelling agent is incorporated into the serum:plasma separator. The serum:plasma separator containing a thixotropic aid has a thixotropic nature at room temperature and is prevented from flowing during storage. At high temperatures, however, the thixotropic nature of the serum:plasma separator decreases, and the viscosity of the serum;plasma separator also decreases. Therefore, the separation aid flows. The serum:plasma separator containing an organic gelling agent is prevented from flowing at both room temperature and at high temperatures. However, the viscosity of the serum:plasma separator increases with time and the performance as a serum:plasma separator decreases.

It is an object of the present invention to provide a serum:plasma separator which prevents hemocytes from remaining in a serum portion after blood separation and prevents migration of a component having a concentration gradient between clot and serum even when separated blood components are stored in a separation tube, whereby correct examination results can be obtained.

It is another object of the present invention to provide a serum:plasma separator which hardly undergoes a form change at room temperature and under heat and which hardly shows a viscosity change even after a long period of time.

It is a further object of the present invention to provide a serum:plasma separator which has excellent thixotropic nature free from a change in temperature, excellent cohesion and excellent adhesion to a blood separation tube.

It is a still further object of the present invention to provide a blood separation tube containing the above serum:plasma separator.

According to the present invention, there is provided a serum:plasma separator having a specific gravity, measured at 25°C, of 1.035 to 1.060 and obtainable by adding from 0.1 to 60 parts by weight of a tackifier which is a thermoplastic having a softening point of from 5 to 150°C and a weight average molecular weight of from 200 to 1500 to 100 parts by weight of a partition-forming material which is an oily polymer.

According to the present invention, there is also provided a tube for the separation of serum:plasma from clot:hemocyte, prepared by placing a separator according to the present invention in a one end-closed tube and closing the other end.

The serum:plasma separator of the present invention is obtained from the partition-forming material and the tackifier and optionally, a lipophilic laminar inorganic compound. Thus, the partition-forming material may further comprise a lipophilic laminar inorganic compound. The separator has a specific gravity of 1.035 to 1.060 in the intermediate region between the specific gravity of the serum and that of the clot or between the specific gravity of the plasma and that of the hemocytes. The above-defined specific gravity can be achieved by properly selecting the partition-forming material, the tackifier and, optionally, the lipophilic laminar inorganic compound. A large difference in specific gravity between clot or hemocytes and the serum:plasma separator is preferred, since the larger the difference in the specific gravity, the greater is the floatability of the serum;plasma separator in centrifugal separation. However, when the serum:plasma separator has a specific gravity of less than 1.035, undesirably, part of the serum:plasma separator is sometimes separated into the serum or plasma after the centrifugal separation.

The partition-forming material used in the present invention is an oily polymer, preferably having a viscosity, measured at 25°C, of 0.2 to 600 Pa.s (200 to 600,000 centipoises (cps)). Such oily polymers can be properly used to achieve proper flowability and proper gelation in practical use. Examples of these oily polymers include silicone, chlorinated polybutene, chlorinated polybutadiene, poly(meth)acrylate, polyisobutene and a copolymer obtained from an α-olefin or styrene and maleic acid diester.

The tackifier used in the serum:plasma separator of the present invention is a thermoplastic which is a solid or a semi-solid at room temperature. It has a softening point of from 5 to 150°C and a weight average molecular weight of from 200 to 1,500. When incorporated into an oily polymer, the tackifier improves the adhesion and wettability to a blood separation tube.

A tackifier suitable for use in the separator of the invention may be a tackifier derived from a natural resin or a tackifier formed of a synthetic resin. The tackifier is an additive which is added to a polymer to impart the polymer with tackiness.

Examples of the tackifiers derived from natural resins include rosin, dammar, polymerized rosin, partially hydrogenated rosin, glycerin ester rosin, partially hydrogenated glycerin ester rosin, completely hydrogenated glycerin ester rosin, polymerized glycerin ester rosin, pentaerythritol ester rosin, partially hydrogenated pentaerythritol ester rosin, polymerized pentaerythritol ester rosin, α-pinene, a polymer of β-pinene, polyterpene resins such as a dipentene polymer, hydrogenated polyterpene resins, and terpene phenol.

Examples of the tackifiers formed of synthetic resins include olefin and diolefin polymers, a cyclopentadiene resin, an aromatic petroleum resin, a hydrogenation product of an aromatic resin, a phenolic resin, an alkyl phenol-acetylene resin, a styrene resin, a xylene resin, a coumarone indene resin, and a vinyl toluene-a-methylstyrene copolymer resin.

The amount of the tackifier for use in the present invention varies depending upon the compatibility with the partition-forming material and its viscosity. The amount of the tackifier per 100 parts by weight of the partition-forming material is 0.1 to 60 parts by weight, preferably 1 to 30 parts by weight.

When the amount of the tackifier is less than the above lower limit, the cohesion of the serum;plasma separator and the adhesion of the serum:plasma separator to a blood separation tube are insufficient. When a lipophilic laminar inorganic compound is also incorporated, the amount of the tackifier adsorbed on the lipophilic laminar inorganic compound is too small to obtain sufficient thixotropic nature. When the amount of the tackifier is more than the above upper limit, the compatibility between the tackifier and the partition-forming material is deteriorated, causing a phase separation between these two components, or the viscosity of the serum:plasma separator increases to make the operation of centrifugal separation difficult.

The lipophilic laminar inorganic compound is typically prepared by replacing interlayer metal ions of clay mineral, such as natural or synthetic mica, bentonite or smectite, with an onium compound having a lipophilic group, such as an alkyl ammonium salt. The lipophilic laminar inorganic compound easily swells by taking an organic compound having a small or intermediate size into interlayer spaces. As a result, the serum:plasma separator has high thixotropy. The oil absorption properties of the lipophilic laminar inorganic compound change with temperature, and the change in the thixotropic nature of the serum:plasma separator with temperature change can be minimized.

The amount of the lipophilic laminar inorganic compound for use in the serum:plasma separator is determined according to the viscosity and specific gravity of the serum:plasma separator. The amount of lipophilic laminar inorganic compound, when present, is preferably 0.1 to 5 parts by weight per 100 parts by weight of the partition-forming material. When the amount of the lipophilic laminar inorganic compound is too small, sufficient thixotropic nature cannot be imparted to the serum:plasma separator, and flowing of the serum;plasma separator is caused. When this amount is too large, the serum:plasma separator shows an increase in the viscosity and specific gravity and is not suitable for practical use.

The serum:plasma separator of the present invention has the following physical properties. That is, the specific gravity measured at 25°C is between the specific gravity of serum and that of clot or between the specific gravity of plasma and that of hemocyte, i.e., 1.035 to 1.060. The separator typically has a viscosity measured at 25°C, of 200 to 2000 Pa.s (200,000 to 2,000,000 centipoises).

The serum:plasma separator of the present invention may further contain an inorganic powder, an organic additive and a blood coagulant. Examples of the inorganic powder include silica, talc and alumina. Examples of the organic additive include wax, a plasticizer and a gelling agent.

The serum:plasma separator of the present invention can be produced by heating the partition-forming material up to 80 to 200°C, adding a predetermined amount of the tackifier and optionally a predetermined amount of the lipophilic laminar inorganic compound, and either stirring the resultant mixture under heat or preparing a dispersion of the resultant mixture with a roll.

The serum:plasma separator of the present invention exhibits smaller flowing with time at room temperature and under heat and a smaller change in viscosity with time than any conventional serum:plasma separator which is prepared from an inorganic powder or an organic gelling agent but does not contain a tackifier in combination. The reasons for these advantages are that the serum:plasma separator of the present invention has, unlike a conventional serum:plasma separator, thixotropic nature free from a change in temperature, cohesion and adhesion to a tube. As a result, the serum:plasma separator of the present invention hardly changes its form with time even if its viscosity is not high, and the viscosity of the serum:plasma separator of the present invention hardly changes with time.

The present invention will be detailed hereinafter by reference to Examples, in which "part" stands for "part by weight".

### Example 1

A four-necked flask was charged with 100 parts of a copolymer (viscosity at 25°C: 2 Pa.s (2,000 centipoises)) obtained from a mixture of an α-olefin having 12 carbon atoms with an *α*-olefin having 14 carbon atoms and dimethyl maleate (α-olefins/dimethyl maleate molar ratio was 1/1) and 40 parts of pentaerythritol ester of partially hydrogenated rosin (Pentalyn H, supplied by Rika Hercules, PENTALYN is a trade mark), and the resultant mixture was stirred at 100°C for 2 hours to give a serum separator having a viscosity of 2,000 Pa.s (2,000,000 centipoises) and a specific gravity of 1.035. One gram of the serum separator was placed in a 15 cm³ (cc) test tube, and the test tube was tightly closed with a butyl rubber closure and vacuumed. Then, 10 cm³ of a blood sample was also placed in the test tube. Then, the test tube was allowed to stand for 1 day, and then the blood was centrifugally separated at 1,200 g for 10 minutes. The result was that the serum separator was positioned between clot and serum and that no hemocyte remained in the serum. When the test tube was stored for 7 days as it contained the separated blood components, no migration of components from the hemocytes to the serum was observed.

The viscosity values described in this Example and those in Examples below were obtained by measurement with an E-type viscometer.

### Example 2

A four-necked flask was charged with 100 parts of a copolymer (viscosity at 25°C: 2 Pa.s (2,000 centipoises)) obtained from a mixture of an α-olefin having 12 carbon atoms with an α-olefin having 14 carbon atoms and dimethyl maleate (α-olefins/dimethyl maleate molar ratio was 1/1)and 10 parts of pentaerythritol ester of partially hydrogenated rosin (Pentalyn H, supplied by Rika Hercules), the resultant mixture was stirred at 100°C for 1 hour. The so-obtained mixture and 2 parts of alkylammonium-modified natural smectite (Benton 38, supplied by NL Industry, BENTON is a Trade mark) were kneaded with a three-roll mill to give a serum separator having a viscosity of 400 Pa.s (400,000 centipoises) and a specific gravity of 1.040. One gram of the serum separator was placed in a 15 cm³ test tube, and the test tube was tightly closed with a butyl rubber closure and vacuumed. Then, 10 cm³ of a blood sample was also placed in the test tube. Then, the test tube was allowed to stand for 1 day, and then the blood was centrifugally separated at 1,200 g for 10 minutes. The result was that the serum separator was positioned between clot and serum and that no hemocyte remained in the serum. When the test tube was stored for 7 days as it contained the separated blood components, no migration of components from the hemocytes to the serum was observed. Further, 1.5 g of the above-obtained serum separator was placed in the bottom of a 15 cm³ test tube, and the test tube was placed horizontally in a dryer and stored there at 60°C for 4 days. The result was that the flow length of the serum separator was not more than 2 mm.

### Example 3

A four-necked flask was charged with 100 parts of polyisobutene having a number average molecular weight of about 1,000 and a viscosity of 1.5 Pa.s (1,500 centipoises) (measured at 25°C) and 5 parts of glycerin ester of partially hydrogenated rosin (Staybelite 10, supplied by Rika Hercules, STAYBELITE is a trade mark), and the resultant mixture was stirred at 100°C for 1 hour. The so-obtained mixture and 2 parts of a silica powder were kneaded with a three-roll mill to give a serum separator having a viscosity of 400 Pa.s (400,000 centipoises) and a specific gravity of 1.040. One gram of the serum separator was placed in a 15 cm³ test tube, and the test tube was tightly closed with a butyl rubber closure and vacuumed. Then, 10 cm³ of a blood sample was also placed in the test tube. Then, the test tube was allowed to stand for 1 day, and then the blood was centrifugally separated at 1,200 g for 10 minutes. The result was that the serum separator was positioned between clot and serum and that no hemocyte remained in the serum. When the test tube was stored for 7 days as it contained the separated blood components, no migration of components from the hemocytes to the serum was observed. Further, 1.5 g of the above-obtained serum separator was placed in the bottom of a 15 cm³ test tube, and the test tube was placed horizontally in a dryer and stored there at 60°C for 4 days. The result was that the flow length of the serum separator was not more than 2 mm.

### Example 4

A four-necked flask was charged with 100 parts of polyisobutene having a number average molecular weight of about 1,000 and a viscosity of 1.5 Pa.s (1,500 centipoises) (measured at 25°C) and 5 parts of glycerin ester of partially hydrogenated rosin (Staybelite 10, supplied by Rika Hercules), and the resultant mixture was stirred at 100°C for 1 hour. The so-obtained mixture and 3 parts of alkylammonium-modified natural smectite (Benton 38, supplied by NL Industry) were kneaded with a three-roll mill to give a serum separator having a viscosity of 400 Pa.s (400,000 centipoise) and a specific gravity of 1.048. One gram of the serum separator was placed in a 15 cm³ test tube, and the test tube was tightly closed with a butyl rubber closure and vacuumed. Then, 10 cm³ of a blood sample was also placed in the test tube. Then, the test tube was allowed to stand for 1 day, and then the blood was centrifugally separated at 1,200 g for 10 minutes. The result was that the serum separator was positioned between clot and serum and that no hemocyte remained in the serum. When the test tube was stored for 7 days as it contained the separated blood components, no migration of components from the hemocytes to the serum was observed. Further, 1.5 g of the above-obtained serum separator was placed in the bottom of a 15 cm³ test tube, and the test tube was placed horizontally in a dryer and stored there at 60°C for 4 days. The result was that the flow length of the serum separator was not more than 2 mm.

### Example 5

100 Parts of a copolymer obtained from a mixture of an α-olefin having 12 carbon atoms with an α-olefin having 14 carbon atoms and dimethyl maleate (α-olefins/dimethyl maleate molar ratio was 1/1), 20 parts of a terpentine resin (YS resin Px #1000, supplied by Yasuhara Chemical Co., Ltd.) and 2 parts of alkylammonium-modified natural smectite (Benton 38, supplied by NL Industry) were kneaded with a three-roll mill to give a serum separator having a viscosity of 500 Pa.s (500,000 centipoises) and a specific gravity of 1.042. One gram of the serum separator was placed in a 15 cm³ test tube, and the test tube was tightly closed with a butyl rubber closure and vacuumed. Then, 10 cm³ of a blood sample was also placed in the test tube. Then, the test tube was allowed to stand for 1 day, and then the blood was centrifugally separated at 1,200 g for 10 minutes. The result was that the serum separator was positioned between clot and serum and that no hemocyte remained in the serum. When t.he test tube was stored for 7 days as it contained the separated blood components, no migration of components from the hemocytes to the serum was observed. Further, 1.5 g of the above-obtained serum separator was placed in the bottom of a 15 cm³ test tube, and the test tube was placed horizontally in a dryer and stored there at 60°C for 4 days. The result was that the flow length of the serum separator was not more than 1 mm.

### Comparative Example 1

One gram of a copolymer (viscosity at 25°C: 2 Pa.s (2,000 centipoises)) obtained from a mixture of an α-olefin having 12 carbon atoms with an α-olefin having 14 carbon atoms and dimethyl maleate (α-olefins/dimethyl maleate molar ratio was 1/1) was placed in a 15 cm³ test tube, and the test tube was tightly closed with a butyl rubber closure and vacuumed. Then, 10 cm³ of a blood sample was also placed in the test tube. The test tube was allowed to stand for 1 day, and then the blood was centrifugally separated at 1,200 g for 10 minutes. The result was that the serum separator was positioned between clot and serum but that no hemocyte remained in the serum. When the test tube was stored for 7 days as it contained the separated blood components, migration of components from the hemocytes to the serum was observed. Further, 1.5 g of the above-obtained serum separator was placed in the bottom of a 15 cm³ test tube, and the test tube was placed horizontally in a dryer and stored there at 60°C for 1 hour. The result was that almost all the serum separator flowed.

### Comparative Example 2

100 Parts of a copolymer (viscosity at 25°C: 2 Pa.s 2,000 centipoises) obtained from a mixture of an α-olefin having 12 carbon atoms with an α-olefin having 14 carbon atoms and dimethyl maleate (α-olefins/dimethyl maleate molar ratio was 1/1), 2.2 parts of alkylammonium-modified natural smectite (Benton 38, supplied by NL Industry) and 0.5 part of colloidal silica were kneaded with a three-roll mill to give a serum separator having a viscosity of 600 Pa.s (600,000 centipoises) and a specific gravity of 1.045. One gram of the serum separator was placed in a 15 cm³ test tube, and the test tube was tightly closed with a butyl rubber closure and vacuumed. Then, 10 cm³ of a blood sample was also placed in the test tube. Then, the test tube was allowed to stand for 1 day, and then the blood was centrifugally separated at 1,200 g for 10 minutes. The result was that the serum separator was positioned between clot and serum but that hemocytes remained in the serum. When the test tube was stored for 7 days as it contained the separated blood components, migration of components from the hemocytes to the serum was observed. Further, 1.5 g of the above-obtained serum separator was placed in the bottom of a 15 cm³ test tube, and the test tube was placed horizontally in a dryer and stored there at 60°C for 1 day. The result was that the flow length of the serum separator was 8 mm.

### Comparative Example 3

100 Parts of a copolymer (viscosity at 25°C: 2 Pa.s 2,000 centipoises) obtained from a mixture of an α-olefin having 12 carbon atoms with an α-olefin having 14 carbon atoms and dimethyl maleate (α-olefins/dimethyl maleate molar ratio was 1/1) and 0.5 part of a condensate obtained from sorbitol and benzaldehyde (Gelol D, supplied by Shin-Nippon Rikasha) were kneaded with a three roll mill to give a serum separator having a specific gravity of 1.050 and a viscosity of 500 Pa.s (500,000 centipoises) 1.5 Grams of the serum separator was placed in the bottom of a 15 cm³ test tube, and the test tube was tightly closed with a butyl rubber closure and vacuumed. Then, the test tube was placed horizontally in a dryer and stored there at 60°C for 1 day to show that the flow length of the serum separator was not more than 1 mm. However, the viscosity of the serum separator increased with time, and after one year, the serum separator did not flow at all even in centrifugal separation.

## Claims

1. A serum:plasma separator having a specific gravity, measured at 25°C, of 1.035 to 1.060 and obtainable by adding from 0.1 to 60 parts by weight of a tackifier which is a thermoplastic having a softening point of from 5 to 150°C and a weight average molecular weight of from 200 to 1500 to 100 parts by weight of a partition-forming material which is an oily polymer.

2. A separator according to claim 1, wherein the amount of tackifier is 1 to 30 parts by weight per 100 parts by weight of the partition-forming material.

3. A separator according to claim 1 or 2, wherein the partition-forming material further comprises a lipophilic laminar inorganic compound.

4. A separator according to claim 3, wherein the amount of lipophilic laminar inorganic compound is 0.1 to 5 parts by weight per 100 parts by weight of the partition-forming material.

5. A separator according to claim 3 or 4, wherein the lipophilic laminar inorganic compound is a product obtainable by replacing interlayer metal ions of clay mineral with an onium compound having a lipophilic group.

6. A separator according to any one of the preceding claims which has a viscosity, measured at 25°C, of 200 to 2000 Pa.s (200,000 to 2,000,000 centipoises).

7. A separator according to any one of the preceding claims wherein the partition-forming material is an oily polymer having a viscosity, measured at 25°C, of 0.2 to 600 Pa.s (200 to 600,000 centipoises).

8. A separator according to any one of the preceding claims wherein the tackifier is a tackifier derived from a natural resin or a tackifier formed of a synthetic resin.

9. A tube for the separation of serum:plasma from clot:hemocytes, prepared by placing a separator as claimed in any one of the preceding claims in a one end-closed tube and closing the other end.

## Patentansprüche

1. Serum:Plasma-Separator, welcher eine relative Dichte, gemessen bei 25°C, von 1,035 bis 1,060 aufweist und erhältlich ist durch Zugeben von 0,1 bis 60 Gewichtsteilen cines Klebrigmachers, welcher ein Thermoplast mit einem Erweichungspunkt von 5 bis 150°C und einer gewichtsmittleren Molekülmasse von 200 bis 1 500 ist, zu 100 Gewichtsteilen eines eine Verteilung bildenden bzw. Auftrennung hervorrufenden Materials, welches ein öliges Polymer ist.

2. Separator nach Anspruch 1, worin die Menge des Klebrigmachers 1 bis 30 Gewichtsteile pro 100 Gewichtsteile des eine Verteilung bildenden Materials beträgt.

3. Separator nach Anspruch 1 oder 2, worin das eine Verteilung bildende Material ferner eine lipophile, laminare, anorganische Verbindung umfaßt.

4. Separator nach Anspruch 3, worin die Menge der lipophilen, laminaren anorganischen Verbindung 0,1 bis 5 Gewichtsteile pro 100 Gewichtsteile des eine Verteilung bildenden Materials beträgt.

5. Separator nach Anspruch 3 oder 4, worin die lipophile, laminare, anorganische Verbindung ein Produkt ist, das durch Ersetzen von Zwischenschicht-Metallionen von Ton-Mineral mit einer Oniumverbindung mit einer lipophilen Gruppe erhältlich ist.

6. Separator nach einem der vorstehenden Ansprüche, welcher eine Viskosität, gemessen bei 25°C, von 200 bis 2 000 Pa·s (200 000 bis 2 000 000 Centipoise) besitzt.

7. Separator nach einem der vorstehenden Ansprüche, worin das eine Verteilung bildende Material ein öliges Polymer mit einer viskosität, gemessen bei 25°C, von 0,2 bis 600 Pa·s (200 bis 600 000 Centipoise) ist.

8. Separator nach einem der vorstehenden Ansprüche, worin der Klebrigmacher ein aus einem natürlichen Harz abgeleiteter Klebrigmacher oder ein aus einem synthetischen Harz gebildeter Klebrigmacher ist.

9. Rohr zur Separation von Serum:Plasma aus Blutgerinnsel:Blutzellen, hergestellt durch Einbringen eines, wie in einem beliebigen der vorstehenden Ansprüche beanspruchten Separators in ein an einem Ende verschlossenes Rohr und Verschließen des anderen Endes.

## Revendications

1. Séparateur de sérum:plasma ayant une densité, mesurée à 25°C, comprise entre 1,035 et 1,060 et pouvant être obtenu en ajoutant de 0,1 à 60 parties en poids d'un agent collant, qui est une matière thermoplastique ayant un point de ramollissement compris entre 5 et 150°C et un poids moléculaire moyen en poids compris entre 200 et 1500 à 100 parties en poids d'un matériau formant séparation, qui est un polymère huileux.

2. Séparateur selon la revendication 1, dans lequel la quantité d'agent collant est comprise entre 1 et 30 parties en poids pour 100 parties en poids du matériau formant séparation.

3. Séparateur selon la revendication 1 ou la revendication 2, dans lequel le matériau formant séparation comprend en outre un composé inorganique laminaire lipophile.

4. Séparateur selon la revendication 3, dans lequel la quantité de composé inorganique laminaire lipophile est comprise entre 0,1 et 5 parties en poids pour 100 parties en poids du matériau formant séparation.

5. Séparateur selon la revendication 3 ou la revendication 4, dans lequel le composé inorganique laminaire lipophile est un produit pouvant être obtenu en remplaçant les ions métalliques intersticiels d'un minéral argileux par un composé onium ayant un groupe lipophile.

6. Séparateur selon l'une quelconque des revendications précédentes, qui a une viscosité, mesurée à 25°C, comprise entre 200 et 2000 Pa.s (200 000 à 2 000 000 centipoises).

7. Séparateur selon l'une quelconque des revendications précédentes, dans lequel le matériau formant séparation est un polymère huileux ayant une viscosité, mesurée à 25°C, comprise entre 0,2 et 600 Pa.s (200 à 600 000 centipoises).

8. Séparateur selon l'une quelconque des revendications précédentes, dans lequel l'agent collant est un agent collant dérivé d'une résine naturelle ou un agent collant formé d'une résine synthétique.

9. Tube pour la séparation de sérum:plasma de coagulum: hémocytes, préparé en plaçant un séparateur selon l'une quelconque des revendications précédentes dans un tube fermé à une extrémité et en fermant l'autre extrémité.
